Europäisches Patentamt

European Patent Office

Office européen des brevets

(11) Veröffentlichungsnummer: **0 085 276**

**A1**

(12) **EUROPÄISCHE PATENTANMELDUNG**

(21) Anmeldenummer: 82810515.5

(22) Anmeldetag: 01.12.82

(51) Int. Cl.³: **G 01 N 33/58**
**C 12 Q 1/00**

(30) Priorität: **10.12.81 CH 7882/81**

(43) Veröffentlichungstag der Anmeldung:
**10.08.83 Patentblatt 83/32**

(84) Benannte Vertragsstaaten:
**AT BE CH DE FR GB IT LI LU NL SE**

(71) Anmelder: **GREINER ELECTRONICS AG**
**Gaswerkstrasse 33**
**CH-4900 Langenthal(CH)**

(72) Erfinder: **Lauterburg, Rainer**
**Hubackerweg 37**
**CH-4153 Reinach(CH)**

(54) Verfahren zur immunchemischen Bestimmung einer Substanz.

(57) Bei dem Verfahren spielen sich im wesentlichen drei Inkubationen ab, unterbrochen von nicht dargestellten Reinigungsvorgängen.

Ein an eine feste Phase gebundenes Reagenz, z.B. eim Antikörper (Ab 1), wird mit der ein gesuchtes Antigen (Ag) enthaltenden Probe zusammen einer ersten Inkubation (I) unterworfen. Die nicht gebundenen Probenteile werden entfernt und die feste Phase (1) mit einer Peroxidase-Lösung zusammengebracht. Letztere enthält Peroxidase (POD), welche an einen Antikörper (Ab 2) gebunden ist, der ebenfalls für das gesuchte Antigen (Ag) spezifisch, aber meistens vom Antikörper (Ab 1) verschieden ist. Während dieser zweiten Inkubation (II) wird Peroxidase (POD) indirekt und nach Massgabe des zu bestimmenden Antigens (Ag) über dieses an die feste Phase (1) gebunden. Schliesslich wird freie oder gebundene Peroxidase (POD) in einer dritten Inkubation (III) dazu herangezogen, um in Gegenwart von Wasserstoffperoxid $H_2O_2$ aus einer fluorierten phenolischen Verbindung R-F eine entsprechende Menge von Fluorid-Ionen $F^-$ freizusetzen, welche ihrerseits elektrochemisch ionenselektiv bestimmt werden.

Fig. 1

## Verfahren zur immunchemischen Bestimmung einer Substanz

Die vorliegende Erfindung befasst sich mit einem Verfahren gemäss dem Oberbegriff des Patentanspruches 1. Bei derartigen Verfahren ergibt die immunchemische Reaktion der gesuchten Substanz entsprechende Mengen gebundener oder freier Reaktionspartner mit Markierstoffen, welche durch radioaktive oder optische Messverfahren erfassbar sind. Obschon diese Messverfahren teilweise sehr empfindlich und/oder selektiv sind, werden die dazu erforderlichen Apparaturen meistens ziemlich aufwendig in Anschaffung oder Anwendung.

Es ist Aufgabe der Erfindung, ein Verfahren zu schaffen, welches die erwähnten Nachteile vermindert oder behebt. Sie ist im Kennzeichen des Patentanspruches 1 umschrieben, vorteilhafte Weiterbildungen in den weiteren Ansprüchen.

Durch die Erfindung werden die umständlichen Vorkehrungen optischer Verfahren, aber auch die Gefahren der radioaktiven Markierung vermieden, kinetische Messungen erleichtert. Weitere Vorteile ergeben sich aus der nachfolgenden Beschreibung eines Ausführungsbeispiels. In den Zeichnungen zeigen

Fig. 1  ein immunchemisches Schema des Verfahrens

Fig. 2  eine Elektroden-Messzelle in Aufriss-Schnitt

Fig. 3  dieselbe Zelle in Grundriss-Schnitt längs Ebene A-A in Fig. 2

Fig. 4  einen vergrösserten Ausschnitt aus Fig. 3

Fig. 5  ein Magazin für Reagenz-Träger in Ansicht

Fig. 6  dasselbe Magazin in Draufsicht

./.

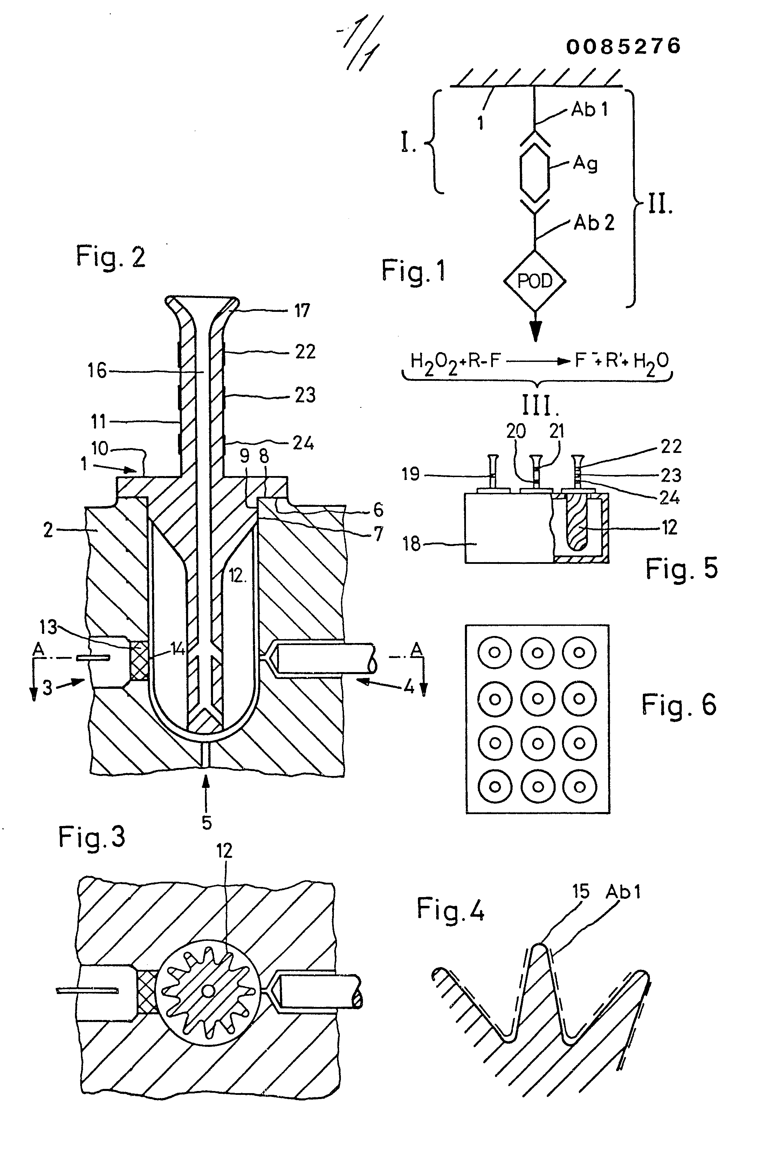

Nach dem Schema Fig.1 spielen sich bei dem erfindungsgemässen Verfahren im wesentlichen drei Inkubationen ab, unterbrochen von nicht dargestellten Reinigungsvorgängen. Ein an eine feste Phase gebundenes Reagenz, z.B. der Antikörper Ab 1 , wird mit der ein gesuchtes Antigen Ag enthaltenden Probe zusammen einer ersten Inkubation I unterworfen. Die nicht gebundenen Probenteile werden entfernt und die feste Phase 1 mit einer Peroxidase-Lösung zusammengebracht. Letztere enthält Peroxidase POD , welche an einen Antikörper Ab 2 gebunden ist, der ebenfalls für das gesuchte Antigen Ag spezifisch, aber meistens vom Antikörper Ab 1 verschieden ist. Während dieser zweiten Inkubation II wird Peroxidase POD indirekt und nach Massgabe des zu bestimmenden Antigens Ag über dieses an die feste Phase 1 gebunden. Schliesslich wird freie oder gebundene Peroxidase POD in einer dritten Inkubation III dazu herangezogen, um in Gegenwart von Wasserstoffperoxid $H_2O_2$ aus einer fluorierten phenolischen Verbindung R-F eine entsprechende Menge von Fluorid-Ionen $F^-$ freizusetzen, welche ihrerseits elektrochemisch ionenselektiv bestimmt werden.

Dieses allgemeine Verfahren verbindet die Vorteile des Enzym-Immuno-Assays (EIA) nach der sog. "Sandwich-Methode" mit denjenigen der Fluorid-Elektrode, insbesondere deren ausserordentlichen Selektivität, wodurch dem EIA weitere Anwendungsgebiete erschlossen werden. Andere EIAs als die "Sandwich-Methode" kommen, sofern sie POD-Komplexe aufweisen, ebenfalls in Frage.

Eine einfache Einrichtung zur Durchführung des Verfahrens gemäss der Erfindung weist nach der Fig.2 einen Träger 1 für die feste Phase des Immunsystems auf, der im Gefäss 2 eingesetzt ist. Dieses Gefäss 2 weist die für Elektroden-Messzellen übliche Ausrüstung auf, nämlich eine Mess-Elektrode 3, eine Bezugselektroden-Anordnung 4 und ein Abflussleitungssystem 5, die alle nur teilweise veranschaulicht sind. Ausserdem besitzt das Gefäss 2 gemäss der Erfindung Lagerflächen 6,7, welche mit entsprechenden Lagerflächen 8,9 am Deckel 10 des Trägers 1 zusammenwirken. Von der Mitte des Deckels 10 ragt ein Stiel 11 nach oben, und nach unten erstrecken sich eine Anzahl radiale Rührflügel 12 ins Gefäss 2, siehe Fig.3. Der als Membran der Elektrode 3 dienende Lanthan-Trifluorid-Kristall 13 ist derart geformt und im Körper des Gefässes 2 eingekittet, und die Rührflügel 12 derart ausgebildet, dass sich dazwischen ein nur schmaler Rührspalt 14 ergibt, (aus zeichnerischen Gründen in Fig. 2 und 3 zu gross dargestellt.) Auf diese Weise ist Gewähr geboten, dass durch Drehen des Trägers 1, z.B. am Stiel 11 oder Deckel 10 stets frische Flüssigkeit vor die Elektrodenmembran 13 gefördert und so die hohe Messgenauigkeit aufrecht erhalten wird.

Um auch die Konstanz der am Träger 1 angelagerten Reagenzmenge zu gewährleisten, sind gemäss Fig.4 die dem Rührspalt 14 nächstgelegenen Partien 15 der Rührflügel 12 nicht mit Reagenz Ab 1 versehen.

Damit bei Bedarf auch Flüssigkeit in das Gefäss 2 zu-oder abgeführt werden kann, ohne das Abfluss-System 5 zu öffnen, oder bei Fehlen desselben, ist der Träger 1 mit einer zentralen Bohrung 16, und wenn nötig mit einem kleinen Trichter 17 versehen. Die Ausflussöffnungen unten an der Bohrung 16 können dem Zweck entsprechend optimiert werden, wie überhaupt die Formgebung des Trägers 1 und des Gefässes 2 den jeweiligen Anforderungen angepasst werden kann. Dies gilt insbesondere dann, wenn das erfindungsgemässe Verfahren automatisiert werden soll.

Zweckmässigerweise werden die verschiedenartigen Träger 1 gemäss Fig. 5 und 6 in Magazinen 18 in Reihen angeordnet, welch jede z.B. einer anderen zu bestimmenden Substanz entspricht, bzw. einem anderen Antigen Ag . Als

./.

Arbeitserleichterung und um Verwechslungen zu vermeiden, können die Träger 1 auch mit Kennungen 19 bis 24 versehen sein, welche auch maschinenlesbar sein können.

Die immunchemische Bestimmung einer Substanz kann gemäss der Erfindung im einzelnen wie folgt durchgeführt werden:

Ein für das gesuchte Antigen Ag spezifisch mit Antikörpern versehender Träger 1 wird dem Magazin 18 entnommen und in das Gefäss 2 eingesetzt, in welches bereits die allenfalls verdünnte Probenflüssigkeit Ag dosiert worden war. Durch hin- und herdrehen am Trägerstiel 11 wird schnell ein Gleichgewicht der Immunreaktion erzielt, d.h. alles spezifische Proben-Antigen Ag hat sich an den im Ueberschuss vorhandenen Träger-Antikörpern Ag1 gebunden. Die restliche Probenflüssigkeit wird entfernt, und Träger 1 und Gefäss 2 werden gründlich gewaschen. Hierauf wird Peroxidase POD zugeführt, welche an spezifische Antikörper Ab 2 gebunden und gegenüber dem Antigen Ag im Ueberschuss vorhanden ist. Während der Träger 1 hin- und her gedreht wird, findet ein genau dem Proben-Antigen Ag entsprechender Teil der Peroxidase-Komplexe eine Bindung an das letztere. Wiederum wird gewaschen wie vorher und hernach das Gefäss 2 mit Wasserstoffperoxyd $H_2O_2$ u.Fluorophenol R-F beschickt. Nun wird wiederum am Träger 1 gedreht und die ionenselektive Messung der entstehenden Fluorionen in Gang gesetzt. An Hand von Eichkurven wird schliesslich der Gehalt der Probe an dem gesuchten Antigen Ag bestimmt. Die Eichkurven werden aufgestellt, indem man die genau gleichen Messmethoden statt auf eine unbekannte Probe Ag auf Antigenlösungen mit abgestuften bekannten Gehalten, die Eich- oder Standardlösungen, anwendet.

./.

Bei dem soeben beschriebenen Verfahren ist der Aufwand an Gerätschaften sehr klein, indem neben dem Mess- und dem Waschgefäss keine weiteren Gefässe benötigt werden. Das Waschgefäss kann unter Umständen auch noch weggelassen und der Träger 1 von Anfang bis Ende der Prozedur im Messgefäss 2 belassen werden, dann nämlich, wenn auch das Waschen im Letzteren erfolgt, z.B. durch die Bohrung 16 hindurch. Eine solche Minimalapparatur könnte sogar den Arzt bei Hausbesuchen begleiten und ihm trotzdem sehr genaue Ergebnisse in relativ kurzer Zeit liefern.

Umgekehrt ist, wie schon oben erwähnt, ein Ausbau in Richtung Automatisierung sämtlicher Hantierungen möglich. Schliesslich sind auch die immunchemischen Vorgänge verschiedener Abwandlungen im Rahmen der Erfindung fähig, z.B. auch Austausch der Funktionen von Antigen und Antikörper, und es sind auch andere ionensensitive Elektroden als die beschriebenen möglich, sowie auch z.B. Sauerstoffelektroden.

Solche Varianten der Erfindung werden aber hier um der gewünschten Kürze willen nicht im Einzelnen beschrieben.

Patentansprüche

1. Verfahren zur immunchemischen Bestimmung einer Substanz in einer flüssigen Probe (Ag), welche man mit einem an eine feste Phase (1) gebundenen Reagenz (Ab 1) zur immunchemischen Reaktion bringt, woraufhin das Ergebnis über eine Enzymmarkierung gemessen wird, dadurch gekennzeichnet, dass durch die Reaktion die Menge entstehender oder vorhandener Ionen beeinflusst wird, und dass die Messung mittels einer ionenselektiven Elektrode (3) erfolgt.

2. Verfahren nach Anspruch 1, dadurch gekennzeichnet, dass die Flüssigkeit (Ag) mittels eines Rührgliedes (12) derart bewegt wird, dass sie sowohl am Träger (1) der festen Phase als auch an der Elektrode (3) vorbeibewegt wird.

3. Verfahren nach Anspruch 2, dadurch gekennzeichnet, dass ein Träger (1) verwendet wird, welcher zugleich als Rührglied (12) und/oder als Mundstück (16) für die Zufuhr der Probe dienen kann.

4. Verfahren nach Anspruch 1, dadurch gekennzeichnet, dass man zur Bestimmung eines immunchemischen Reaktionspartners (Ag) diesen einerseits an einen festen Träger (1) und anderseits an Peroxidase (POD) bindet, dass man hernach Wasserstoffperoxid ($H_2O_2$) und ein Fluorophenol (R-F) beigibt, und dass man die durch das gebundene oder das freie Peroxid (POD) katalisierte Spaltung des Fluorophenols (R-F) beobachtet, indem man die dabei entstehenden Fluorid-Ionen ($F^-$) mittels einer für die letzteren selektiven Elektrode (3) misst.

5. Verfahren nach Anspruch 4, dadurch gekennzeichnet, dass zur Bestimmung eines mehrwertigen Antigens (Ag) die Peroxidase (POD) über eine "Sandwich-Immunbindung" vom Typ "Peroxidase-Antikörper (Ab2)/Antigen (Ag)/Antikörper (Ab1)-Träger (1)" an den Träger (1) gebunden wird, wobei die Bestimmung wie folgt abläuft:

a) Einführen des mit Antikörper (Ab1) versehenen Trägers (1) und Dosieren der Probe (Ag) in ein Reaktionsgefäss und dadurch Beginn einer ersten Inkubation (I), mit selektiver Kopplung des Antigens (Ag) mit dem Antikörper (Ab1) am Träger (1);

b) Entfernen der restlichen Probenflüssigkeit von Reaktionsgefäss und Träger (1);

c) Zuführen einer Peroxidaselösung , welche Peroxidase (POD)-Antikörper (Ab2)-Komplexe im Ueberschuss enthält, und dadurch Beginn einer zweiten Inkubation (II);

d) Ueberführen, entweder des Trägers (1) mit der gebundenen Peroxidase (POD), oder der Rest-Peroxidase-Lösung mit der frei beweglichen Peroxidase (POD), in ein Messgefäss (2), und Beifügen der Wasserstoffperoxid ($H_2O_2$)- und der Fluorophenol (R-F)-Lösungen zum Messgefäss (2), welches die Fluoridelektrode (3) enthält, wodurch sich eine dritte Inkubation (III) ergibt;

e) Beobachten der von letzterer abgegebenen elektrischen Spannung als Mass für die Menge des zu bestimmenden Proben-Antigens (Ag).

6. Einrichtung zur Durchführung des Verfahrens nach Anspruch 1, gekennzeichnet durch ein Gefäss (2) für die Probenflüssigkeit (Ag) in Verbindung mit einer festen Phase (1) und daran haftendem immunchemischen Reagenz (Ab1) und mit einer ionenselektiven Elektrode (3).

7. Einrichtung nach Patentanspruch 6, dadurch gekennzeichnet, dass die feste Phase (1) an einem als Rührglied (12) ausgebildeten festen Träger (1) angebracht ist, dass dieser Träger (1) durch Lagerflächen (6 bis 9) in Bezug auf das Gefäss (2) derart bewegbar gelagert und die Elektrode (3) derart im Gefäss (2) befestigt sind, dass zwischen Rührglied (12) und Elektrodenkopf (13) ein nur schmaler Rührspalt (14) für die Flüssigkeit (Ag) frei bleibt.

./.

8. Einrichtung nach Anspruch 7, dadurch gekennzeichnet, dass an die dem Rührspalt (14) benachbarte Partie (15) des Rührgliedes (12) kein Reagenz (Ab1) angelagert ist.

9. Einrichtung nach Anspruch 7, dadurch gekennzeichnet, dass der Träger (1) im Gefäss (1) rotierbar gelagert ist, achsparallel oder spiralig nach abwärts und nach aussen gerichtete Rührflügel (12), einen Deckel (10) und einen von dessen Mitte aus aufragenden Stiel (11) aufweist.

10. Einrichtung nach Anspruch 9, dadurch gekennzeichnet, dass sich durch Stiel (11) und Deckel (10) eine Bohrung (16) abwärts bis in den Raum zwischen den Rührflügeln (12) erstreckt, welche zum Einfüllen und/oder Entleeren von Flüssigkeiten beim Gefäss (2) benützbar ist.

0085276

Fig. 1

Fig. 2

Fig. 3

Fig. 4

Fig. 5

Fig. 6

$$H_2O_2 + R\text{-}F \longrightarrow F^- + R' + H_2O$$

Europäisches
Patentamt

**EUROPÄISCHER RECHERCHENBERICHT**

**0085276**
Nummer der Anmeldung

EP  82 81 0515

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X,P | ANALYTICAL CHEMISTRY, Band 54, Nr. 1, Januar 1982, Easton, P.W. ALEXANDER et al. "Enzyme-linked immunoassay of human immunoglobulin G with the fluoride ion selective electrode", Seiten 68-71<br>* Zusammenfassung * | 1 | G 01 N   33/58<br>C 12 Q    1/00 |
| A | WO-A-8 001 081  (BATTELLE MEMORIAL INSTITUTE)<br>* Zusammenfassung * | 1,4 | |
| A | ARZNEIMITTEL-FORSCHUNG, Band 28, Nr. 11A, 1978, Aulendorf, L. SCHROTT "Enzymimmunoassay", Seiten 1934-1940 | | **RECHERCHIERTE SACHGEBIETE (Int. Cl. ³)** |
| A | DE-A-3 010 462  (DR. E. FRESENIUS)<br>* Figur; Seite 7, Zeilen 1-6 * | 1 | C 12 Q    1/00<br>G 01 N   33/58 |
| A | DE-A-2 824 742  (R.N. PIASIO et al.)<br>* Figur 1; Anspruch 6 * | 6,7,8 | |

Der vorliegende Recherchenbericht wurde für alle Patentansprüche erstellt.

| Recherchenort<br>BERLIN | Abschlußdatum der Recherche<br>04-03-1983 | Prüfer<br>SCHWARTZ K |
|---|---|---|